(19) Europäisches Patentamt

**European Patent Office**

Office européen des brevets

(11) Publication number: **0 469 477 B1**

# (12) EUROPEAN PATENT SPECIFICATION

(45) Date of publication of patent specification: **20.09.95**

(21) Application number: **91112577.1**

(22) Date of filing: **26.07.91**

(51) Int. Cl.6: **A61K 31/55**, A61K 31/44,
//(A61K31/55,31:425),
(A61K31/44,31:425)

(54) **Antiallergic combination.**

(30) Priority: **02.08.90 US 561743**

(43) Date of publication of application:
**05.02.92 Bulletin 92/06**

(45) Publication of the grant of the patent:
**20.09.95 Bulletin 95/38**

(84) Designated Contracting States:
**AT BE CH DE DK FR GB IT LI LU NL SE**

(56) References cited:
**EP-A- 0 345 931**

**CHEMICAL ABSTRACTS, vol. 110, no. 21, 22
May 1989, Columbus, OH (US); R. PATTER-
SON et al., p. 586, no. 190981f**

(73) Proprietor: **F. HOFFMANN-LA ROCHE AG
Postfach 3255
CH-4002 Basel (CH)**

(72) Inventor: **O'Donnell, Margaret
148 Allwood Road,
Unit 4
Clifton, N.J. 07014 (US)**
Inventor: **Welton, Ann
37 Maple Drive
North Caldwell, N.J. 07006 (US)**

(74) Representative: **Kellenberger, Marcus, Dr. et
al
Grenzacherstrasse 124
Postfach 3255
CH-4002 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to compositions comprising the synergistic combination of platelet activating factor (PAF) antagonists with leukotriene $D_4$ ($LTD_4$) antagonists and the use thereof to treat allergic reactions.

The mechanism of pathogenesis associated with allergic reactions involves an antigen-induced release of a variety of allergic mediators from mast cells, basophils, and possibly macrophages. These mediators include histamine, leukotrienes and PAF all of which possess a broad spectrum of potent biological activities [Schleimer, R.P., Am. Rev. Respir. Dis., **133**, 614-617 (1986) and Barnes, P.J., J. Allergy Clin. Immunol., **81**,152-160 (1988)]. Assessment of the precise contribution of each individual allergic mediator to the pathology of allergic reactions is unknown.

For example, allergic asthma involves the antigen-induced release of allergic mediators from mast cells which subsequently cause bronchoconstriction, mucus secretion, and changes in capillary permeability which, in combination with the production of chemotactic agents, leads to the influx into the lung of inflammatory cells. The mediators rapidly induce a narrowing and occluding of the bronchial lumen of the airways and, over time, the development of a chronic inflammatory condition in the lungs. Inflammation of the airways may be essential to the establishment of bronchial hyperresponsiveness which is the characteristic abnormality of asthma [Chung, K. F., Thorax, **41**, 657-662 (1986)]. It has been difficult to identify the predominant mediator(s) of allergic asthma, but in recent years a number of lines of evidence strengthen the concept that the bronchoactive peptidoleukotrienes ($LTC_4$, $LTD_4$ and $LTE_4$ ) and PAF may be important in this disease process. $LTD_4$ and PAF have been identified in body fluids during an episode of asthma They are both potent mediators of inflammation, and can reproduce some of the pathophysiological features of the disease when administered to animals and man [O'Donnell, M. *in* Therapeutic Approaches to Inflammatory Diseases, A. J. Lewis, N. S. Doherty and N. R. Ackerman, eds., Elsevier Science Publishing Co., New York, 169-193 (1989)].

Compounds which prevent the effects of the mediators are thus of interest in treating allergic reactions. However, since allergic reactions are complex processes that are probably not attributable to a single causative factor, a potent, specific, agent by itself may not be particularly effective in preventing allergic reactions.

Int Arch Allergy Appl Immunol 1989; 88; 462-470 describes a combination of 2 receptor antagonists to leukotriene $D_4$ (LTD4) and platelet-activating factor PAF). This combination of anti $LTD_4$ and anti PAF was shown to inhibit airway responses to either $LTD_4$ or PAF in separate experiments in Ascaris airway-reactive animals.

Surprisingly, it has now been found that protection against allergic reactions such as antigen-induced death in mammals can be achieved by administering to such mammals an effective amount of a composition comprising a synergistic combination of a PAF antagonist and a $LTD_4$ antagonist.

In accordance with the invention, there can be used as the PAF antagonist component of a composition of the invention a known compound selected from the group consisting of:

5-{3-[4-(2-chlorophenyl)-9-methyl-6H-thieno[3,2-f][1,2,4]triazolo[4,3-a]   [1,4]diazepin-2-yl]-2-propynyl}phenanthridin-6(5H)-one;

(R)-(2E,4E)-5-(4-methoxyphenyl)-N-[1-methyl-4-(3-pyridinyl)butyl]-2,4-decadienamide;

3-[4-[(R)-2-[(methoxycarbonyl)oxy]-3-[(octadecylcarbamoyl)oxy]propoxy] butyl]thiazolium iodide;

rac-4-{[6-(2-chlorophenyl)-8,9-dihydro-1-methyl-4H,7H-cyclopenta [4,5]thieno [3,2-f][1,2,4]triazolo[4,3-a][1,4]-diazepin-8-yl]carbonyl}morpholine;

rac-N,N-dipropyl-6-{[6-(2-chlorophenyl)-8,9-dihydro-1-methyl-4H,7H-cyclopenta[4,5]thieno[3,2-f][1,2,4]-triazolo[4,3-a][1,4]diazepine-8-carboxamide;

rac-6-(2-chlorophenyl)-8,9-dihydro-1-methyl-8-(4-morpholinyl)methyl-4H,7H-cyclopenta   [4,5]thieno[3,2-f]-[1,2,4]triazolo[4,3-a][1,4]diazepine;

4-(2-chlorophenyl)-9-methyl-2-{2-[4-(2-methylpropyl)phenyl]ethyl}-6H-thieno[3,2-f]  [1,2,4]triazolo[4,3-a][1,4]-diazepine;

4-(2-chlorophenyl)-6,9-dimethyl-2-{2-[4-(2-methylpropyl)phenyl]ethyl}-6H-thieno[3,2-f]   [1,2,4]triazolo[4,3-a]-[1,4]diazepine;

2,3-dihydro-5-{4-[2-(3,4,5-trimethoxyphenyl)ethyl]phenyl}imidazo[2,1-a]isoquinoline;

(2S,5S)-2-[(3-methylsulfonyl-5-methoxy-4-propyloxy)phenyl]-5-(3,4,5-trimethoxyphenyl)tetrahydrofuran;

( + )-2-(2-acetyl-6-methoxy-3,9-dioxo-4,8-dioxa-2,10-diazaoctacos-1-yl)-1-ethyl pyridinium chloride;

( + )-N-(3-benzoylphenyl)-3-(3-pyridinyl)-1H,3H-pyrrolo[1,2-c]thiazole-7-carboxamide;

1-O-hexadecyl-2(R,S)-O-ethyl-3-O-[7-(thiazolio-heptyl)]glycerol mesylate; and

1-acetyl-4-(8-chloro-5,6-dihydro-11H-benzo[5,6]cyclohepta[1,2-b]pyridin-11-ylidene)piperidine.

Preferred PAF antagonists comprise the following compounds:

5-{3-[4-(2-chlorophenyl)-9-methyl-6H-thieno[3,2-f][1,2,4]triazolo[4,3-a][1,4]diazepin-2-yl]-2-propynyl}phenanthridin-6(5H)-one;

(R)-(2E,4E)-5-(4-methoxyphenyl)-N-[1-methyl-4-(3-pyridinyl)butyl]-2,4-decadienamide;

3-[4-[(R)-2-[(methoxycarbonyl)oxy]-3-[(octadecylcarbamoyl)oxy]propoxy]butyl] thiazolium iodide; and

(2S,5S)-2-[(3-methylsulfonyl-5-methoxy-4-propyloxy)phenyl]-5-(3,4,5-trimethoxyphenyl)tetrahydrofuran.

Most preferred PAF antagonists are:

5-{3-[4-(2-chlorophenyl)-9-methyl-6H-thieno[3,2-f][1,2,4]triazolo[4,3-a][1,4]diazepin-2-yl]-2-propynyl}phenanthridin-6(5H)-one; and

(R)-(2E,4E)-5-(4-methoxyphenyl)-N-[1-methyl-4-(3-pyridinyl)butyl]-2,4-decadienamide.

Also, in accordance with the invention, there can be used as the LTD$_4$ antagonist component of a composition a known compound selected from the group consisting of:

(E)-4-[3-[2-(4-cyclobutyl-2-thiazolyl)ethenyl]phenylamino]-2,2-diethyl-4-oxobutanoic acid;

rac-6-acetyl-7-[[5-(4-acetyl-3-hydroxy-2-propylphenoxy)pentyl]oxy]-3,4-dihydro-2H-1-benzopyran-2-carboxylic acid;

5-(3-(2-(7-chloroquinolin-2-yl)ethenyl)phenyl)-4,6-dithianonanedicarboxylic acid mono N,N-dimethylamide, sodium salt;

[3-[[2-methoxy-4-[[[(2-methylphenyl)sulfonyl]amino]carbonyl]phenyl]methyl]-1-methyl-1H-indol-5-yl]carbamic acid cyclopentyl ester;

(E)-4-[3-[2-(4-(1-methylethyl)-2-thiazoyl)ethenyl]phenylamino]-2,2-diethyl-4-oxobutanoic acid;

5-[[2-[[4-(2-quinolinylmethoxy)phenoxy]methyl]phenyl]methyl]-1H-tetrazole;

5-(7-(3-(quinolin-2-yl)methoxy)phenylmethoxy)-4-oxo-4H-1-benzopyran-2-yl)-1H-tetrazole;

3(S)-(2-(carboxethyl)thio-3-(2-(8-phenyloctyl)phenyl)propanoic acid;

2(S)-hydroxy-3(R)-(2-(carboxethyl)thio)-3-(2-(8-phenyloctyl)phenyl)propanoic acid;

1-[2-hydroxy-3-propyl-4-[4-[2-[4-(1H-tetrazol-5-yl)butyl]-2H-tetrazol-5-yl] butoxy]phenyl]ethanone;

5-{3-[2(R)-(carboxyethylthio)-1(S)-hydroxypentadeca-3(E), 5(Z)-dienyl] phenyl}-1H-tetrazole

2-(3-(2-quinolylmethoxy)phenylamino)benzoic acid;

1,1,1-trifluoro-N-[3-(2-quinolinylmethoxy)phenyl]methanesulfonamide;

N-[3-[3-(4-acetyl-3-hydroxy-2-propylphenoxy)-propoxy]-4-chloro-6-methylphenyl]-1H-tetrazole-5-carboxamide, sodium salt;

8-[4-(4-phenylbutyloxy)benzoyl]amino-2-(tetrazol-5-yl)-4-oxo-4H-1-benzopyran;

N-[2,3-dihydro-3-(1H-tetrazol-5-yl)-1,4-benzodioxin-5-yl]-4-(4-phenyl butoxy)benzamide;

rac-7-[3-(4-acetyl-3-hydroxy-2-propylphenoxy)propoxy]-3,4-dihydro-2-methyl-4-oxo-8-propyl-2H-1-benzopyran-2-propanoic acid;

[[5-[[3-(4-acetyl-3-hydroxy-2-propylphenoxy)-propyl]thio]-1,3,4-thiadiazol-2-yl]thio] acetic acid;

9-(4-acetyl-3-hydroxy-2-propylphenoxymethyl)-3-((1H)-tetrazol-5-yl)-4H-pyrido[1,2-a]pyrimidin-4-one;

6-(2-cyclohexylethyl)-[1,3,4]-thiadiazolo[3,2-a]-1,2,3-triazolo[4,5-d]pyrimidin-9(1H)-one; and

1-[[2-(1-methylethyl)pyrazolo][1,5-a]pyridin-3-yl]-2-methyl-1-propanone.

Preferred LTD$_4$ antagonists comprise the following compounds:

(E)-4-[3-[2-(4-cyclobutyl-2-thiazolyl)ethenyl]phenylamino]-2,2-diethyl-4-oxobutanoic acid;

rac-6-acetyl-7-[[5-(4-acetyl-3-hydroxy-2-propylphenoxy)pentyl]oxy]-3,4-dihydro-2H-1-benzopyran-2-carboxylic acid; and

5-(3-(2-(7-chloroquinolin-2-yl)ethenyl)phenyl)-4,6-dithianonanedicarboxylic acid mono N,N-dimethylamide, sodium salt.

The most preferred LTD$_4$ antagonist is:

(E)-4-[3-[2-(4-cyclobutyl-2-thiazolyl)ethenyl]phenylamino]-2,2-diethyl-4-oxobutanoic acid.

The synergistic compositions and methods of the invention due to their antiallergic activity, can be utilized for the treatment of allergic disorders such as seasonal rhinitis, perennial vasomotor rhinitis, acute urticaria, chronic urticaria, atopic dermatitis, contact dermatitis, pruritis, angioedema, conjunctivitis, chronic bronchitis, systemic anaphylaxis, serum sickness, bronchial asthma, food allergies, and related inflammatory diseases including inflammatory bowel disease, and psoriasis.

The anti-allergic effect of the compositions of the invention comprising combinations of PAF antagonists and LTD$_4$ antagonists may be demonstrated utilizing procedures described hereinafter.

A. Active Sensitization

Male (Hartley) guinea pigs (200-300 g) are sensitized with an intraperitoneal injection of ovalbumin (10 mg) in 1 ml of 0.9% w/v NaCl solution (saline) on days 1 and 3. The animals are used for the test on days 20-30 following the ovalbumin injections.

B. Pulmonary and Cardiovascular Mechanics

The sensitized guinea pigs are anesthetized with an intraperitoneal injection of urethane (2 g/kg), and polyethylene cannulae are introduced into both jugular veins and the right carotid artery. The arterial catheter is connnected to a pressure transducer for measurement to systemic blood pressure. The venous catheters are used for injection and infusion of the test composition.

The trachea is cannulated through an anterior cervical incision and positive pressure ventilation is applied with a Harvard pump set at a stroke volume of 4.5 ml and a rate of 40 breaths per minute. The respiratory muscles are paralyzed by periodic injections of succinylcholine chloride (1.2 mg/kg) given intravenously.

To measure transpulmonary pressure, a 12 gauge hypodermic needle attached via a short length of Tygon tubing to one side of a Statham differential pressure transducer is introduced through the fifth or sixth intercostal space into the intrapleural space near the sternum, leaving a pneumothorax. The other side of this transducer is connected to the side-arm of the tracheal cannula by a second Tygon tube of identical length and bore as the first. Airflow is measured with a Fleisch pneumotachograph head connected to a tracheal cannula and a second Statham differential pressure transducer. Electrical integration of the flow signal provides a recording of tidal volume.

Pulmonary resistance and dynamic lung compliance are calculated manually from the transpulmonary pressure, airflow, and volume signals as described by Amdur and Mead and the results are used to calibrate an online analog pulmonary computer [Am. J. Physiol.,**192**,364-368 (1958)]. Blood pressure is recorded with a cannula inserted into the carotid artery. Heart rate is calculated by an on-line analog cardiovascular computer. All six parameters (airflow, transpulmonary pressure, tidal volume, resistance, compliance and blood pressure) are recorded simultaneously on a polygraph recorder and these parameters, plus heart rate and respiratory rate, are printed out on an electronic data terminal. Alter completion of surgery, a period of no less than 5 minutes is allowed for cardiopulmonary functions to stabilize. During this period, control values are recorded continuously.

C. Challenge

Following surgical preparation, the animals are pretreated with intravenous indomethacin (10 mg/kg) 22 minutes before ovalbumin challenge presumably to shunt arachidonic acid from the cyclooxygenase pathway into the lipoxygenase pathway for the formation of leukotrienes, and to eliminate the modulating effects of prostaglandins. Spontaneous breathing is arrested with succinylcholine (1.2 mg/kg, i.v.) 7 minutes before antigen challenge. Treatment with succinylcholine results in a constant baseline ventilatory pressure of 6-8 cm $H_2O$. The bronchoconstrictor effects of histamine are abolished with pyrilamine (2.0 mg/kg, i.v.), administered 6 minutes before antigen challenge. To potentiate the bronchoconstrictor effect of antigen, propranolol (0.1 mg/kg, i.v.) is administered before antigen challenge. [Anderson, W. H., *et al.*, Br. J. Pharmacol.,**78**,67-74 (1983)].

Various antagonists or control vehicle are administered intravenously through a cannula in the jugular vein 5 minutes before the animals are challenged with a maximum constrictory dose of ovalbumin (1 mg/kg) given intravenously.

The percent change in pulmonary resistance, dynamic lung compliance, and the systemic blood pressure and survival time are monitored for 120 minutes.

RESULTS

When the aforementioned model of anaphylaxis procedure was utilized to test various combinations of the invention, animals administered vehicle alone and challenged with antigen exhibited bronchoconstriction (approximately 550% increase in pulmonary resistance and 80% decrease in dynamic lung compliance), hypotension (55 mmHg decrease), and 83% mortality within 120 minutes. Table I contains the results obtained in this model with various combinations of the invention.

## TABLE I

### PROTECTION OF ANAPHYLACTIC DEATH IN SENSITIZED GUINEA PIG BY THE SYNERGISTIC COMPOSITIONS OF THE INVENTION

| COMPOUNDS | DOSE mg/kg i.v. | COMPOUND | % SURVIVAL at 120 min. |
|---|---|---|---|
| ALONE * | | | |
| Control Vehicle | | | 17 |
| A | ( 1 mg/kg) | PAF antagonist | 0 |
| B | ( 1 mg/kg) | $LTD_4$ antagonist | 0 |
| C | ( 1 mg/kg) | $LTD_4$ antagonist | 50 |
| D | (10 mg/kg) | $LTD_4$ antagonist | 0 |
| E | ( 1 mg/kg) | PAF antagonist | 33 |
| F | ( 1 mg/kg) | PAF antagonist | 0 |
| G | ( 1 mg/kg | PAF antagonist | 50 |
| COMBINATIONS OF THE INVENTION * | | | |
| A+B | | | 100 |
| A+C | | | 100 |
| A+D | | | 100 |
| B+E | | | 100 |
| B+F | | | 100 |
| B+G | | | 100 |
| C+G | | | 100 |

\*    COMPOUNDS

**A =** 5-{3-[4-(2-Chlorophenyl)-9-methyl-6H-thieno[3,2-f]1,2,4]triazolo-[4,3-a][1,4]diazepin-2-yl]-2-propynyl}phenanthridin-6(5H)-one.

**B =** (E)-4-[3-[2-(4-Cyclobutyl-2-thiazolyl)ethenyl]phenylamino]-2,2-diethyl-4-oxobutanoic acid.

**C =** 5-(3-(2-(7-Chloroquinolin-2-yl)ethenyl)phenyl)-4,6-dithianonane dicarboxylic acid, mono N,N-dimethylamide, sodium salt.

**D =** rac-6-Acetyl-7-[[5-(4-acetyl-3-hydroxy-2-propylphenoxy)pentyl]oxy]-3,4-dihydro-2H-1-benzopyran-2-carboxylic acid.

**E =** (R)-(2E,4E)-5-(4-Methoxyphenyl)-N-[1-methyl-4-(3-pyridinyl)butyl]-2,4-decadienamide.

**F =** 3-[4-[(R)-2-[(Methoxycarbonyl)oxy]-3-[(octadecylcarbamoyl)oxy]propoxy]-butyl]thiazolium iodide.

**G =** (2S,5S)-2-[(3-Methylsulfonyl-5-methoxy-4-propyloxy)phenyl]-5-(3,4,5-trimethoxyphenyl)tetrahydrofuran.

The ability of the $LTD_4$ antagonist, compound B [(E)-4-[3-[2-(4-cyclobutyl-2-thiazolyl)ethenyl]-phenylamino]-2,2-diethyl-4-oxobutanoic acid] and the PAF antagonist, compound A [5-{3-[4-(2-chlorophenyl)-9-methyl-6H-thieno[3,2-f][1,2,4]triazolo[4,3-a][1,4]diazepin-2-yl]-2-propynyl}phenanthridin-6-(5H)-one] to block antigen-induced bronchospasm, hypotension and death was evaluated at doses (1.0 mg/kg, i.v., 5 minute pretreatment) previously shown to completely inhibit bronchoconstriction induced by exogenous $LTD_4$ or PAF, respectively. Animals treated with compound B alone had a 0% survival rate at 120 minutes, whereas when the combination was given, there was 100% survival at 120 minutes.

The ability of the $LTD_4$ antagonist, compound C [5-(3-(2-(7-chloroquinolin-2-yl)ethenyl)phenyl) -4, 6-dithianonanedicarboxylic acid, mono N,N-dimethylamide, sodium salt] and the PAF antagonist, compound A to block antigen-induced bronchospasm, hypotension and death was evaluated at doses (1.0 mg/kg, i.v., 5 minute pretreatment) previously shown to completely inhibit bronchoconstriction induced by exogenous $LTD_4$ or PAF, respectively. Animals treated with compound C alone had a 50% survival rate at 120 minutes and those treated with compound A alone had a 0% survival rate at 120 minutes. However, when the combination was given, there was 100% survival at 120 minutes.

The ability of the $LTD_4$ antagonist, compound D [rac-6-acetyl-7-[[5-(4-acetyl-3-hydroxy-2-propylphenoxy)pentyl]oxy]-3,4-dihydro-2H-1-benzopyran-2-carboxylic acid] and the PAF antagonist, compound A to block antigen-induced bronchospasm, hypotension and death was evaluated at doses (10 mg/kg and 1.0 mg/kg, i.v., 5 minute pretreatment, respectively) previously shown to completely inhibit bronchoconstriction induced by exogenous $LTD_4$ or PAF, respectively. Animals treated with compound D or compound A alone had a 0% survival rate at 120 minutes, whereas when the combination was given, there was 100% survival at 120 minutes.

The ability of the $LTD_4$ antagonist, compound B and the PAF antagonist, compound E [(R) - (2E,4E) -5- (4-methoxyphenyl) -N- [1-methyl-4-(3-pyridinyl)butyl]-2,4-decadienamide] to block antigen-induced bronchospasm, hypotension and death was evaluated at doses (1.0 mg/kg, i.v., 5 minute pretreatment) previously shown to completely inhibit bronchoconstriction induced by exogenous $LTD_4$ or PAF, respec-tively. Animals treated with compound B alone had a 0% survival rate at 120 minutes and those treated with compound E alone had a 33% survival rate at 120 minutes. However, when the combination was given, there was 100% survival at 120 minutes.

The ability of the $LTD_4$ antagonist, compound B and the PAF antagonist, compund F [3-[4-[(R)-2-[-(methoxycarbonyl)oxy]-3-[(octadecylcarbamoyl) oxy] propoxy] butyl] thiazolium iodide] to block antigen-induced bronchospasm, hypotension and death was evaluated at doses (1.0 mg/kg, i.v., 5 minute pretreatment) previously shown to completely inhibit bronchoconstriction induced by exogenous $LTD_4$ or PAF, respectively. Animals treated with compound B alone had a 0% survival rate at 120 minutes and those treated with compound F alone had a 0% survival rate at 120 minutes. However, when the combination was given, there was 100% survival at 120 minutes.

The ability of the $LTD_4$ antagonist, compound B and the PAF antagonist, compound G [(2S,5S)-2-[(3-methylsulfonyl-5-methoxy-4-propyloxy)phenyl]-5-(3,4,5-trimethoxyphenyl)tetrahydrofuran] to block antigen-induced bronchospasm, hypotension and death was evaluated at doses (1.0 mg/kg, i.v., 5 minute pretreatment) previously shown to completely inhibit bronchoconstriction induced by exogenous $LTD_4$ or PAF, respectively. Animals treated with compound B alone had a 0% survival rate at 120 minutes and those treated with compound G alone had a 50% survival rate at 120 minutes. However, when the combination was given, there was 100% survival at 120 minutes.

The ability of the $LTD_4$ antagonist, compound C and the PAF antagonist, compound G to block antigen-induced bronchospasm, hypotension and death was evaluated at doses (1.0 mg/kg, i.v., 5 minute pretreatment) previously shown to completely inhibit bronchoconstriction induced by exogenous $LTD_4$ or PAF, respectively. Animals treated with compound C alone had a 50% survival rate at 120 minutes and those treated with compound G alone had a 50% survival rate at 120 minutes. However, when the combination was given, there was 100% survival at 120 minutes.

The compositions of the invention may be administered by any of the modes by which the individual components may be administered, for example, orally, intravenously or the like. The dosage regiment may be regulated according to the potency of individual compounds employed, the mode of administration, and the needs of the host mammal depending on factors such as the degree and the severity of the disease state and age and general condition of the host mammal being treated.

The compositions of the invention can be administered in unit dosage forms such as tablets, capsules, pills, powders, granules, sterile parenteral solutions or suspensions, suppositories, transdermal compositions, aerosols and the like. Such dosage forms are prepared according to standard techniques in the art.

The dosages may be varied depending upon the requirements of the host, the severity of the condition being treated and the particular compound being employed in the composition. Determination of the proper dosage for a particular administration is within the skill of the art. Generally, treatment is initiated at lower dosages and increased as needed by small increments until the optimum effect is reached. Exemplary dosages are in the range of from 0.1 to 4.0 g per day of a combination of the invention. Exemplary dosages of the PAF antagonist can be in the range of 0.01 to 2.0 g per day. Exemplary dosages of the $LTD_4$ antagonist can be in the range of 0.1 to 2.0 g per day. Generally, the PAF antagonist is present in a ratio of from 0.01 to 50 parts to one part of the $LTD_4$ antagonist, preferably in a ratio of from 0.1 to 10 parts to one part of the $LTD_4$ antagonist. For convenience, the total daily dosage may be administered in divided doses.

EXAMPLARY FORMULATIONS COMPRISING THE INVENTION FOLLOW:

EXAMPLE 1

| TABLET FORMULATION | | | |
|---|---|---|---|
| Item | Ingredients | mg/Tablet | |
| 1 | Compound A [1] | 0.1 mg | 250 mg |
| 2 | Compound B [1] | 1.0 mg | 250 mg |
| 3 | Croscarmellose Sodium* | 6.4 mg | 30 mg |
| 4 | Lactose | 100 mg | 255 mg |
| 5 | Avicel PH 101® | 20 mg | 60 mg |
| 6 | Methocel E15**® | 1.5 mg | 9 mg |
| 7 | Magnesium Stearate | 1.0 mg | 6 mg |
| | | 130 mg | 860 mg |

Method of Preparation:

1. Mix Items 1, 2, 3, 4, and 5 in a suitable mixer.

2. Granulate it with aqueous solution of Methocel E15®.

3. Dry granulation at 45-50°C.

4. Pass the granulation through a suitable mill.

5. Add Item 7 and mix.

6. Compress the granulation on a suitable tablet press.

\* Other disintegrants, such as starch derivatives, Cross-Povidone, Amberlite®, or similar in action
may be used alone or in combination to obtain desired disintegration characteristics.
\*\* Binders, such as pregelatinized starch or polyvinyl-pyrrolidone, etc., may be used to obtain a
desired binding. Wetting agents, such as Tweens®, spans, or sodium lauryl sulfate may be added
to obtain desired disintegration and dissolution characteristics.
(1) As used hereinafter-
Compound A is 5-{3-[4-(2-chlorophenyl)-9-methyl-6H-thieno[3,2-f]
[1,2,4]triazolo[4,3-a][1,4]diazepin-2-yl]-2-propynyl} phenanthridin-6(5H)-one, and
Compound B is (E)-4-[3-[2-(4-cyclobutyl-2-thiazolyl)ethenyl]phenyl amino]-2,2-diethyl-4-oxobutanoic acid.

EXAMPLE 2

| CAPSULE FORMULATION | | | |
|---|---|---|---|
| Item | Ingredients | mg/Capsule | |
| 1 | Compound A | 0.1 mg | 250 mg |
| 2 | Compound B | 1.0 mg | 250 mg |
| 3 | Pregelatinized Starch | 5 mg | 20 mg |
| 4 | Modified Starch | 5 mg | 20 mg |
| 5 | Talc | 4.4 mg | 20 mg |
| 6 | Lactose | 84 mg | 88 mg |
| 7 | Magnesium Stearate | 0.5 mg | 2 mg |
| | | 100 mg | 650 mg |

Method of Preparation:

1. Mix Items 1, 2, 3, 4, and 6 and wet granulate with water.

2. Dry granulation at 45-50 °C.

3. Mill through suitable screen using appropriate milling equipment.

4. Add Items 5 and 7 and mix for 5 minutes.

5. Fill into suitable capsules.

EXAMPLE 3

| SOFT GELATIN CAPSULE FORMULATION | | | |
|---|---|---|---|
| Item | Ingredients | mg/ml | |
| 1 | Compound A | 0.1 mg | 250 mg |
| 2 | Compound B | 1.0 mg | 250 mg |
| 3 | Ethoxylated Fatty Acids | 50 mg | 250 mg |
| 4 | Modified Starch | 100 mg | 100 mg |
| 5 | Talc | 1.0 ml | 1.0 ml |

Method of Preparation:

1. Add and mix Items 1 and 2 with Items 3 and 5.

2. Add Item 4 to the material from Step 1 and mix.

3. Add vegetable oil to the required amount.

4. Fill into a suitable capsule.

EXAMPLE 4

| ORAL SOLUTION FORMULATION | | | |
|---|---|---|---|
| Item | Ingredients | mg/5 ml | |
| 1 | Compound A | 0.1 mg | 100 mg |
| 2 | Compound B | 1.0 mg | 100 mg |
| 3 | Alcohol, Anhydrous | 10 mg | 500 mg |
| 4 | PVP K-30 | 10 mg | 500 mg |
| 5 | Aq. Sod. Hydroxide Solution | 1.0 mg | 100 mg |
| 6 | Sucrose | q.s. | q.s. |
| 7 | Flavoring Agent | q.s. | q.s. |
| 8 | PEG-400 q.s. to | 5.0 ml | 5.0 ml |

Method of Preparation:

1. Dissolve Items 4, 6, and 7 in alcohol and PEG-400.

2. Add Items 1 and 2 and mix thoroughly.

3. Add Item 5 to material from Step 2 and mix until Items 1 and 2 dissolve.

4. Add PEG-400 to the required volume.

5. Fill the solution into a suitable container.

EXAMPLE 5

| PARENTERAL FORMULATION (for I. V. or IM use) | | | |
|---|---|---|---|
| Item | Ingredients | mg/ml | |
| 1 | Compound A | 0.1 mg | 10 mg |
| 2 | Compound B | 1.0 mg | 10 mg |
| 3 | Benzyl Alcohol | 10 mg | 20 mg |
| 4 | Propylene Glycol* | 0.4 ml | 0.5 ml |
| 5 | Emulphor EL620**® | 10 mg | 50 mg |
| 6 | Lactic Acid | 0.01 ml | 0.05 ml |
| 7 | Sod. Hydroxide pH to | 6-7 | 6-7 |
| 8 | Water for Injection q.s. to | 1.0 ml | 1.0 ml |

Method for Preparation:

1. Dissolve Items 3, 4, 5, and 6 in water for injection.

2. Add and dissolve Items 1 and 2 in the solution from Step 1.

3. Adjust pH if necessary.

4. Add water for injection to the required volume.

* Solvents or solubilizers, such as polyethylene glycols, alcohol, glycerine, Povidone, lecithin, sorbitan monooleate, or trioleate. Polysorbate 80 or 20 may be used in combination or alone to achieve the adequate solubility and stabilization.
** Powder for reconstitution may be formulated for appropriate solubilization or stabilization.

EXAMPLE 6

| INHALATION AEROSOL FORMULATION (Suspension) | | |
|---|---|---|
| Item | Ingredients | % w/w |
| 1 | Compound A, Micronized | 0.01 |
| 2 | Compound B, Micronized | 0.1 |
| 3 | Sorbitan Trioleate | 0.5 |
| 4 | Freon 11® | 19.39 |
| 5 | Freon 12® | 80 |
| | | Total 100% |

Method of Preparation:

1. Mix Items 1, 2, 3 and 4 and homogenize thoroughly.

2. Fill the concentrate suspension from Step 1 into a suitable can, place in valve, and crimp to seal container.

3. Pressure fill Item 5 to containers from Step 2.

EXAMPLE 7

| INHALATION AEROSOL FORMULATION (Solution) | | |
|---|---|---|
| Item | Ingredients | % w/w |
| 1 | Compound A | 0.01 |
| 2 | Compound B | 0.1 |
| 3 | Ethyl Alcohol | 10 |
| 4 | Ascorbic Acid | 0.5 |
| 5 | Freon 114® | 20.0 |
| 6 | Freon 12® | 68.5 |
| | | Total ≈ 100% |

Method of Preparation:

1. Dissolve Items 1, 2, and 4 in Item 3.

2. Fill solution from Step 1 into a suitable glass bottle, place in valve, and crimp to seal container.

3. Pressure fill Items 5 and 6 into the container from Step 2.

**Claims**

1. A pharmaceutical composition comprising synergistically effective amounts of a PAF antagonist and a $LTD_4$ antagonist, said PAF antagonist being selected from the group consisting of:

5-{3-[4-(2-chlorophenyl)-9-methyl-6H-thienol[3,2-f][1,2,4]triazolo[4,3-a] [1,4] diazepin-2-yl]-2-propynyl}phenanthridin-6(5H)-one;

(R)-(2E,4E)-5-(4-methoxyphenyl)-N-[1-methyl-4-(3-pyridinyl)butyl]-2,4-decadienamide;

3-[4-[(R)-2-[(methoxycarbonyl)oxy]-3-[(octadecylcarbamoyl)oxy] propoxy]butyl] thiazolium iodide;

rac-4-{[6-(2-chlorophenyl)-8,9-dihydro-1-methyl-4H,7H-cyclopenta [4,5]thieno[3,2-f][1,2,4]triazolo[4,3-a]-[1,4]diazepin-8-yl]carbonyl} morpholine;

rac-N,N-dipropyl-6-{6-(2-chlorophenyl)-8,9-dihydro-1-methyl-4H,7H-cyclopenta[4,5]thieno[3,2-f][1,2,4]-triazolo[4,3-a][1,4]diazepine-8-carboxamide;

rac-6-(2-chlorophenyl)-8,9-dihydro-1-methyl-8-(4-morpholinyl)methyl-4H,7H-cyclopenta[4,5]thieno[3,2-f]-[1,2,4]triazolo[4,3-a][1,4]diazepine;

4-(2-chlorophenyl)-9-methyl-2-{2-[4-(2-methylpropyl)phenyl]ethyl}-6H-thieno[3,2-f][1,2,4] triazolo[4,3-a]-[1,4]diazepine;

4-(2-chlorophenyl)-6,9-dimethyl-2-{2-[4-(2-methylpropyl)phenyl]ethyl}-6H-thieno[3,2-f][1,2,4] triazolo[4,3-a][1,4]diazepine;

2,3-dihydro-5-{4-[2-(3,4,5-trimethoxyphenyl)ethyl]phenyl}imidazo[2,1-a]isoquinoline;

(2S,5S)-2-[(3-methylsulfonyl-5-methoxy-4-propyloxy)phenyl]-5-(3,4,5-trimethoxyphenyl)tetrahydrofuran;

( + )-2-(2-acetyl-6-methoxy-3,9-dioxo-4,8-dioxa-2,10-diazaoctacos-1-yl)-1-ethyl pyridinium chloride;

( + )-N-(3-benzoylphenyl)-3-(3-pyridinyl)-1H,3H-pyrrolo[1,2-c]thiazole-7-carboxamide;

1-O-hexadecyl-2(R,S)-O-ethyl-3-O-[7-(thiazolio-heptyl)]glycerol mesylate; and

1-acetyl-4-(8-chloro-5,6-dihydro-11H-benzo[5,6]cyclohepta[1,2-b]pyridin-11-ylidene)piperidine,

and said LTD4 antagonist being selected from the group consisting of:

(E)-4-[3-[2-(4-cyclobutyl-2-thiazolyl)ethenyl]phenylamino]-2,2-diethyl-4-oxobutanoic acid;

rac-6-acetyl-7-[[5-(4-acetyl-3-hydroxy-2-propylphenoxy)pentyl]oxy]-3,4-dihydro-2H-1-benzopyran-2-carboxylic acid;

5-(3-(2-(7-chloroquinolin-2-yl)ethenyl)phenyl)-4,6-dithianonane    dicarboxylic    acid    mono    N,N-dimethylamide, sodium salt;

[3-[[2-methoxy-4-[[[(2-methylphenyl)sulfonyl]amino]carbonyl]phenyl]    methyl]-1-methyl-1H-indol-5-yl]-carbamic acid cyclopentyl ester;

(E)-4-[3-[2-(4-(1-methylethyl)-2-thiazoyl)ethenyl]phenylamino]-2,2-diethyl-4-oxobutanoic acid;

5-[[2-[[4-(2-quinolinylmethoxy)phenoxy]methyl]phenyl]methyl]-1H-tetrazole;

5-(7-(3-(quinolin-2-yl)methoxy)phenylmethoxy)-4-oxo-4H-1-benzopyran-2-yl)-1H-tetrazole;

3 (S)-(2-(carboxethyl)thio-3-(2-(8-phenyloctyl)phenyl)propanoic acid;

2(S)-hydroxy-3(R)-(2-(carboxethyl)thio)-3-(2-(8-phenyloctyl)phenyl) propanoic acid;

1-[2-hydroxy-3-propyl-4-[4-[2-[4-(1H-tetrazol-5-yl)butyl]-2H-tetrazol-5-yl]butoxy] phenyl]ethanone;

5-{3-[2(R)-(carboxyethylthio)-1(S)-hydroxypentadeca-3(E),5(Z)-dienyl] phenyl}-1H-tetrazole;

2-(3-(2-quinolylmethoxy)phenylamino)benzoic acid;

1,1,1-trifluoro-N-[3-(2-quinolinylmethoxy)phenyl]methanesulfonamide;

N-[3-[3-(4-acetyl-3-hydroxy-2-propylphenoxy)-propoxy]-4-chloro-6-methylphenyl]-1H-tetrazole-5-carboxamide, sodium salt;

8-[4-(4-phenylbutyloxy)benzoyl]amino-2-(tetrazol-5-yl)-4-oxo-4H-1-benzopyran;

N-[2,3-dihydro-3-(1H-tetrazol-5-yl)-1,4-benzodioxan-5-yl]-4-(4-phenyl butoxy) benzamide;

rac-7-[3-(4-acetyl-3-hydroxy-2-propylphenoxy)propoxy]-3,4-dihydro-2-methyl-4-oxo-8-propyl-2H-1-benzopyran-2-propanoic acid;

[[5-[[3-(4-acetyl-3-hydroxy-2-propylphenoxy)-propyl]thio]-1,3,4-thiadiazol-2-yl] thio]acetic add;

(9-(4-acetyl-3-hydroxy-2-propylphenoxymethyl)-3-((1H)-tetrazol-5-yl)-4H-pyrido    [1,2-a]pyrimidin-4-one);

6-(2-cyclohexylethyl)-[1,3,4]-thiadiazolo[3,2-a]-1,2,3-triazolo[4,5-d] pyrimidin-9(1H)-one; and

1-[[2-(1-methylethyl)pyrazolo][1,5-a]pyridin-3-yl]-2-methyl-1-propanone,

and an inert pharmaceutical carrier for use as a medicament in the treatment of allergic disorders.

2. A pharmaceutical composition, in accordance with claim 1, wherein the amount of the PAF antagonist is in the range of from 0.01 to 50 parts, to one part of the LTD4 antagonist.

3. A pharmaceutical composition, in accordance with claim 1 or 2, wherein the PAF antagonist is selected from the group consisting of:

5-{3-[4-(2-chlorophenyl)-9-methyl-6H-thieno[3,2-f][1,2,4]triazolo[4,3-a]    [1,4]    diazepin-2-yl]-2-propynyl}phenanthridin-6(5H)-one;

(R)-(2E,4E)-5-(4-methoxyphenyl)-N-[1-methyl-4-(3-pyridinyl)butyl]-2,4-decadienamide;

3-[4-[(R)-2-[(methoxycarbonyl)oxy]-3-[(octadecylcarbamoyl)oxy] propoxy]butyl] thiazolium iodide; and

(2S,5S)-2-[(3-methylsulfonyl-5-methoxy-4-propyloxy)phenyl]-5-(3,4,5-trimethoxyphenyl)tetrahydrofuran,

and the LTD4 antagonist is selected from the group consisting of:

(E)-4-[3-[2-(4-cyclobutyl-2-thiazolyl)ethenyl]phenylamino]-2,2-diethyl-4-oxobutanoic acid;

rac-6-acetyl-7-[[5-(4-acetyl-3-hydroxy-2-propylphenoxy)pentyl]oxy]-3,4-dihydro-2H-1-benzopyran-2-carboxylic acid; and

5-(3-(2-(7-chloroquinolin-2-yl)ethenyl)phenyl)-4,6-dithianonane    dicarboxylic    acid    mono    N,N-dimethylamide, sodium salt.

4. A pharmaceutical composition, in accordance with claim 3, wherein the PAF antagonist is selected from the group consisting of:

5-{3-[4-(2-chlorophenyl)-9-methyl-6H-thieno[3,2-f][1,2,4]triazolo[4,3-a]    [1,4]diazepin-2-yl]-2-propynyl}phenanthridin-6(5H)-one; and

12

(R)-(2E,4E)-5-(4-methoxyphenyl)-N-[1-methyl-4-(3-pyridinyl)butyl]-2,4-decadienamide,
and the LTD$_4$ antagonist is
(E)-4-[3-[2-(4-cyclobutyl-2-thiazolyl)ethenyl]phenylamino]-2,2-diethyl-4-oxobutanoic acid.

5. A pharmaceutical composition, in accordance with claim 4, wherein the PAF antagonist is
5-{3-[4-(2-chlorophenyl)-9-methyl-6H-thieno[3,2-f][1,2,4]triazolo[4,3-a]    [1,4]diazepin-2-yl]-2-propynyl}phenanthridin-6(5H)-one,
and the LTD$_4$ antagonist is
(E)-4-[3-[2-(4-cyclobutyl-2-thiazolyl)ethenyl]phenylamino]-2,2-diethyl-4-oxobutanoic acid.

6. A pharmaceutical composition, in accordance with claim 4, wherein the PAF antagonist is
(R)-(2E,4E)-5-(4-methoxyphenyl)-N-[1-methyl-4-(3-pyridinyl)butyl]-2,4-decadienamide,
and the LTD$_4$ antagonist is
(E)-4-[3-[2-(4-cyclobutyl-2-thiazolyl)ethenyl]phenylamino]-2,2-diethyl-4-oxobutanoic acid.

7. The use of a composition in accordance with any one of claims 1 to 6 for the manufacture of
medicaments against allergic disorders.

## Patentansprüche

1. Pharmazeutische Zusammensetzung, enthaltend synergistisch wirksame Mengen eines PAF-Antagoni-
sten und eines LTD$_4$-Antagonisten, wobei der genannte PAF-Antagonist ausgwählt wird aus einer
Gruppe, bestehend aus
5-{3-[4-(2-Chlorphenyl)-9-methyl-6H-thieno[3,2-f][1,2,4]triazolo[4,3-a][1,4]    diazepin-2-yl]-2-propynyl}phenanthridin-6(5H)-on;
(R)-(2E,4E)-5-(4-Methoxyphenyl)-N-[1-methyl-4-methyl-4-(3-pyridinyl)butyl]-2,4-decadienamid;
3-[4-[(R)-2-[(Methoxycarbonyl)oxy]-3-[(octadecylcarbamoyl)oxy]propoxy]butyl] thiazolium iodid;
rac-4-{[6-(2-Chlorphenyl)-8,9-dihydro-1-methyl-4H,7H-cyclopenta[4,5]thieno    [3,2-f][1,2,4]triazolo[4,3-a]-
[1,4]diazepin-8-yl]carbonyl}morpholin;
rac-N,N-Dipropyl-6-{[6-(2-chlorphenyl)-8,9-dihydro-1-methyl-4H,7H-cyclopenta[4,5]thieno    [3,2-f][1,2,4]-
triazolo[4,3-a][1,4]diazepin-8-carboxamid;
rac-6-(2-Chlorphenyl)-8,9-dihydro-1-methyl-8-(4-morpholinyl)methyl-4H,7H-cyclopenta[4,5]thieno    [3,2-f]-
[1,2,4]triazolo[4,3-a][1,4]diazepin;
4-(2-Chlorphenyl)-9-methyl-2-{2-[4-(2-methylpropyl)phenyl]ethyl}-6H-thieno    [3,2-f][1,2,4]triazolo[4,3-a]-
[1,4]diazepin;
4-(2-Chlorphenyl)-6,9-dimethyl-2-{2-[4-(2-methylpropyl)phenyl]ethyl}-6H-thieno    [3,2-f][1,2,4]triazolo[4,3-
a][1,4]diazepin;
2,3-Dihydro-5-{4-[2-(3,4,5-trimethoxphenyl)ethyl]phenyl}imidazo-[2,1-a] isochinolin;
(2S,5S)-2-[(3-Methylsulfonyl-5-methoxy-4-propyloxy)phenyl]-5-(3,4,5-trimethoxyphenyl)tetrahydrofuran;
(+)-2-(2-Acetyl-6-methoxy-3,9-dioxo-4,8-dioxa-2,10-diiazaoctacos-1-yl)-1-ethyl pyridinium chlorid;
(+)-N-(3-Benzoylphenyl)-3-(3-pyridinyl)-1H,3H-pyrrolo[1,2-c]thiazole-7-carboxamid;
1-O-Hexadecyl-2(R,S)-O-ethyl-3-O-[7-(thiazolioi-heptyl)]glycerol mesylate; und 1-Acetyl-4-(8-chlor-5,6-
dihydro-11H-benzo[5,6]cycloheptal[1,2-b]pyridin-11-yliden)piperidin, und der genannte LTD$_4$-Antagonist
ausgewählt wird aus einer Gruppe, bestehend aus
(E)-4-[3-[2-((4-Cyclobutyl-2-thiazolyl)ethenyl]phenylamino]-2,2-diethyl-4-oxobutansäure;
rac-6-Acetyl--7-[[5-(4-acetyl-3-hydroxy-2-propylphenoxy)pentyl]oxy]-3,4-dihydro-2H-1-benzopyran-2-
carbonsäure;
5-(3-(2-(7-Chlochinolin-2-yl)ethenyl)phenyl)-4,6-dithianonan dicarbonsäure-mono-N,N-dimethylamid, Natriumalz;
[3-[[2-Methoxy-4-[[[(2-methylphenyl)sulfonyl]amino]phenyl]methyl[-1-methyl-ö1H-indol-5-yl]-
karbamidsäure-cyclopentyl-ester;
(E)-4-[3-[2-(4-(1-Methylethyl)-2-thiazolyl)ethenyl]phenylamino]2,2-diethyl-4-oxobutansäure;
5-[[2-[[4-(2-Chinolinylmethoxy)phenoxy]methyl]phenyl]metehyl]-1H-tetrazol;
5-(7-(3-Chinolin-2-yl)methoxy)phenylmethoxy)-4-oxo-4H-1-benzopyran-2-yl)-1H-tetrazol;
3 (S)-(2-(Carboxyethyl)thio-3-(2-(8-phenyloctyl)phenyl)propansäure;
2(S)-Hydroxy-3(R)-(2-(carboxyethyl)thio)-3-(2-(8-phenyloctyl)phenyl) propansäure;
1-[2-Hydroxy-3-propyl-4-[4-[2-[4-(1H-tetrazol-5-yl)butyl]-2H-tetrazol-5-yl]butoxy] phenyl]ethanon;
5-{3-[2(R)-(Carboxyethylthio)-1(S)-hydroxypentadeca-3(E),5(Z)-dienyl]phenyl}-1H-tetrazol;

2-(3-(2-Chinolylmethoxy)phenylamino)benzoesäure;

1,1,1-Trifluor-N-[3-(2-chinolinylmethoxy)phenyl]methansulfonamid;

N-[3-[3-(4-Acetyl-3-hydroxy-2-propylphenoxy)-propoxy]-4-chlor-6-methylphenyl]-1H-tetrazol-5-carboxyamid, Natriumsalz;

8-[4-(4-Phenylbutyloxy)benzoyl]amino-2-(tetrazol-5-yl)-4-oxo-4H-1-benzopyran;

N-[2,3-Dihydro-3-(1H-tetrazol-5-yl)-1,4-benzodioxin-5-yl]-4-(4-phenyl-butoxy) benzamid;

rac-7-[3-(4-Acetyl-3-hydroxy-2-propylphenoxy)propoxy]-3,4-dihydro-2-methyl-4-oxo-8-propyl-2H-1-benzopyran-2-propansäure;

[[5-[[3-(4-Acetyl-3-hydroxy-2-propylphenoxy)-propyl]thio]1,3,4-thiadiazol-2-yl] thio]essigsäure;

(9-(4-Acetyl-3-hydroxy-2-propylphenoxymethyl)-3-((1H)-tetrazol-5-yl)-4H-pyrido[1,2-a]pyrimidin-4-on);

6-(2-Cyclohexylethyl)-[1,3,4]-thiadiazolo[3,2-a]-1,2,3-triazolo[4,5-d]pyrimidin-9(1H)-on; und

1-[[2-(1-Methylethyl)pyrazolo[]1,5-a]pyridin-3-yl]-2-methyl-1-propanon,

und einen inerten pharmazeutischen Träger zur Verwendung als Medikament für die Behandlung von allergischen Störungen.

2. Pharmazeutische Zusammensetzung gemäss Anspruch 1, worin die Menge des PAF-Antagonsiten zwischen 0,01 und 50 Teilen zu einem Teil des LTD$_4$-Antagonisten variiert.

3. Pharmazeutische Zusammensetzung gemäss den Ansprüchen 1 oder 2, wobei der PAF-Antagonist ausgewählt wird aus einer Gruppe, bestehend aus:

5-{3-[4-(2-Chlorphenyl)-9-methyl-6H-thieno[3,2-f][1,2,4]triazolo[4,3-a][1,4]            diazepin-2-yl]-2-propynyl}phenanthridin-6(5H)-on;

(R)-(2E,4E)-5-(4-Methoxyphenyl)-N-[1-methyl-4-methyl-4-(3-pyridinyl)butyl]-2,4-decadienamid;

3-[4-[(R)-2-[(Methoxycarbonyl)oxy]-3-[(octadecylcarbamoyl)oxy]propoxy]butyl] thiazolium iodid; und

(2S,5S)-2-[(3-Methylsulfonyl-5-methoxy-4-propyloxy)phenyl]-5-(3,4,5-trimethoxyphenyl)tetrahydrofuran,

und der LTD$_4$-Antagonist ausgewählt wird aus einer Gruppe, bestehend aus

(E)-4-[3-[2-((4-Cyclobutyl-2-thiazolyl)ethenyl]phenylamino]-2,2-diethyl-4-oxobutansäure;

rac-6-Acetyl--7-[[5-(4-acetyl-3-hydroxy-2-propylphenoxy)pentyl]oxy]-3,4-dihydro-2H-1-benzopyran-2-carbonsäure; und

5-(3-(2-(7-Chlochinolin-2-yl)ethenyl)phenyl)-4,6-dithianonan-dicarbonsäure-mono-N,N-dimethylamid, Natriumalz.

4. Pharmazeutische Zusammensetzung gemäss Anspruch 3, wobei der PAF-Antagonist ausgewählt wird aus einer Gruppe, bestehend aus

5-{3-[4-(2-Chlorphenyl)-9-methyl-6H-thieno[3,2-f][1,2,4]triazolo[4,3-a][1,4]            diazepin-2-yl]-2-propynyl}phenanthridin-6(5H)-on; und

(R)-(2E,4E)-5-(4-Methoxyphenyl)-N-[1-methyl-4-methyl-4-(3-pyridinyl)butyl]-2,4-decadienamid,

und der LTD$_4$-Antagonist ist

(E)-4-[3-[2-((4-Cyclobutyl-2-thiazolyl)ethenyl]phenylamino]-2,2-diethyl-4-oxobutansäure.

5. Pharmazeutische Zusammensetzung gemäss Anspruch 4, wobei der PAF-Antagonist

5-{3-[4-(2-Chlorphenyl)-9-methyl-6H-thieno[3,2-f][1,2,4]triazolo[4,3-a][1,4]            diazepin-2-yl]-2-propynyl}phenanthridin-6(5H)-on,

und der LTD$_4$-Antagonist

(E)-4-[3-[2-((4-Cyclobutyl-2-thiazolyl)ethenyl]phenylamino]-2,2-diethyl-4-oxobutansäure

bedeuten.

6. Pharmazeutische Zusammensetzung gemäss Anspruch 4, wobei der PAF-Antagonist

(R)-(2E,4E)-5-(4-Methoxyphenyl)-N-[1-methyl-4-methyl-4-(3-pyridinyl)butyl]-2,4-decadienamid,

und der LTD$_4$-Antagonist

(E)-4-[3-[2-((4-Cyclobutyl-2-thiazolyl)ethenyl]phenylamino]-2,2-diethyl-4-oxobutansäure,

bedeuten.

7. Verwendung einer pharmazeutischen Zusammensetzung gemäss einen der Ansprüche 1-6 zur Herstellung eines Medikaments gegen allergische Störungen.

**Revendications**

1. Composition pharmaceutique comprenant des quantités à effet synergique d'un antagoniste du PAF et d'un antagoniste du LTD$_4$, ledit antagoniste du PAF étant choisi parmi l'ensemble comprenant :

5-{3-[4-(2-chlorophényl)-9-méthyl-6H-thiéno[3,2-f][1,2,4]triazolo[4,3-a][1,4]diazépine-2-yl]-2-propynyl}phénanthridine-6(5H)-one ;

(R)-(2E,4E)-5-(4-méthoxyphényl)-N-[1-méthyl-4-(3-pyridinyl)butyl]-2,4-décadiènamide ;

iodure de 3-[4-[(R)-2-[(méthoxycarbonyl)oxy]-3-[(octadécylcarbamoyl)oxypropoxy]butyl]thiazolium ;

rac-4-{[6-(2-chlorophényl)-8,9-dihydro-1-méthyl-4H,7H-cyclopenta[4,5]thiéno[3,2-f][1,2,4]triazolo[4,3-a][1,4]diazépine-8-yl]carbonyl}morpholine ;

rac-N,N-dipropyl-6-{[6-(2-chlorophényl)-8,9-dihydro-1-méthyl-4H,7H-cyclopenta[4,5]thiéno[3,2-f]-[1,2,4]triazolo[4,3-a][1,4]diazépine-8-carboxamide ;

rac-6-(2-chlorophényl)-8,9-dihydro-1-méthyl-8-(4-morpholinyl)méthyl-4H,7H-cyclopenta[4,5]thiéno-[3,2-f][1,2,4]triazolo[4,3-a][1,4]diazépine ;

4-(2-chlorophényl)-9-méthyl-2-{2-[4-(2-méthylpropyl)phényl]éthyl}-6H-thiéno[3,2-f][1,2,4]triazolo[4,3-a][1,4]diazépine ;

4-(2-chlorophényl)-6,9-diméthyl-2-{2-[4-(2-méthylpropyl)phényl]éthyl}-6H-thiéno[3,2-f][1,2,4]triazolo-[4,3-a][1,4]diazépine ;

2,3-dihydro-5-{4-[2-(3,4,5-triméthoxyphényl)éthyl]phényl}imidazo[2,1-a]isoquinoléine ;

(2S,5S)-2-[(3-méthylsulfonyl-5-méthoxy-4-propyloxy)phényl]-5-(3,4,5-triméthoxyphényl)-tétrahydrofuranne ;

chlorure de ( + )-2-(2-acétyl-6-méthoxy-3,9-dioxo-4,8-dioxa-2,10-diazaoctacos-1-yl)-1-éthylpyridinium ;

( + )-N-(3-benzoylphényl)-3-(3-pyridinyl)-1H,3H-pyrrolo[1,2-c]thiazole-7-carboxamide ;

mésylate de 1-O-hexadécyl-2(R,S)-O-éthyl-3-O-[7-(thiazolio-heptyl)]glycérol ; et 1-acétyl-4-(8-chloro-5,6-dihydro-11H-benzo[5,6]cyclohepta[1,2-b]pyridine-11-ylidène)pipéridine,

et ledit antagoniste du LTD$_4$ étant choisi parmi l'ensemble comprenant :

acide (E)-4-[3-[2-(4-cyclobutyl-2-thiazolyl)éthényl]phénylamino]-2,2-diéthyl-4-oxobutanoïque ;

acide rac-6-acétyl-7-[[5-(4-acétyl-3-hydroxy-2-propylphénoxy)pentyl]oxy]-3,4-dihydro-2H-1-benzopyranne-2-carboxylique ;

sel de sodium du mono-N,N-diméthylamide de l'acide 5-(3-(2-(7-chloroquinoléine-2-yl)éthényl)-phényl)-4,6-dithianonanedicarboxylique ;

ester cyclopentylique de l'acide [3-[[2-méthoxy-4-[[[2-méthylphényl)sulfonyl]amino]carbonyl]-phényl]méthyl]-1-méthyl-1H-indole-5-yl]carbamique ;

acide (E)-4-[3-[2-(4-(1-méthyléthyl)-2-thiazoyl)éthényl]phénylamino]-2,2-diéthyl-4-oxobutanoïque ;

5-[[2-[[4-(2-quinoléinylméthoxy)phénoxy]méthyl]phényl]méthyl]-1H-tétrazole ;

5-(7-(3-(quinoléine-2-yl)méthoxy)phénylméthoxy)4-oxo-4H-1-benzopyranne-2-yl)-1H-tétrazole ;

acide 3(S)-2-(carboxyéthyl)thio-3-(2-(8-phényloctyl)phényl)propanoïque ;

acide 2(S)-hydroxy-3(R)-(2-(carboxyéthyl)thio)-3-(2-(8-phényloctyl)phényl)propanoïque ;

1-[2-hydroxy-3-propyl-4-[4-[2-[4-(1H-tétrazole-5-yl)butyl]-2H-tétrazole-5-yl]butoxy]phényl]éthanone ;

5-{3-[2(R)-(carboxyéthylthio)-1(S)-hydroxypentadéca-3(E),5(Z)-diényl]phényl}-1H-tétrazole ;

acide 2-(3-(2-quinoléylméthoxy)phénylamino)benzoïque ;

1,1,1-trifluoro-N-[3-(2-quinoléinylméthoxy)phényl]méthanesulfonamide ;

sel de sodium du N-[3-[3-(4-acétyl-3-hydroxy-2-propylphénoxy)-propoxy]-4-chloro-6-méthylphényl]-1H-tétrazole-5-carboxamide ;

8-[4-(4-phénylbutyloxy)benzoyl]amino-2-(tétrazole-5-yl)-4-oxo-4H-1-benzopyranne ;

N-[2,3-dihydro-3-(1H-tétrazole-5-yl)-1,4-benzodioxine-5-yl]-4-(4-phénylbutoxy)benzamide ;

acide rac-7-[3-(4-acétyl-3-hydroxy-2-propylphénoxy)propoxy]-3,4-dihydro-2-méthyl-4-oxo-8-propyl-2H-1-benzopyranne-2-propanoïque ;

acide [[5-[[3-(4-acétyl-3-hydroxy-2-propylphénoxy)propyl]thio]-1,3,4-thiadiazole-2-yl]thio] acétique ;

9-(4-acétyl-3-hydroxy-2-propylphénoxyméthyl)-3-((1H)-tétrazole-5-yl)-4H-pyrido[1,2-a]pyrimidine-4-one ;

6-(2-cyclohexyléthyl)-[1,3,4]-thiadiazolo[3,2-a]-1,2,3-triazolo[4,5-d]pyrimidine-9(1H)-one ; et

1-[[2-(1-méthyléthyl)pyrazolo][1,5-a]pyridine-3-yl]-2-méthyl-1-propanone,

et un excipient pharmaceutique inerte, pour utilisation comme médicament dans le traitement des troubles allergiques.

2. Composition pharmaceutique selon la revendication 1, dans laquelle la quantité de l'antagoniste du PAF est comprise dans l'intervalle de 0,01 à 50 parties pour 1 partie de l'antagoniste du $LTD_4$.

3. Composition pharmaceutique selon la revendication 1 ou 2, dans laquelle l'antagoniste du PAF est choisi parmi l'ensemble comprenant :

5-{3-[4-(2-chlorophényl)-9-méthyl-6H-thiéno[3,2-f][1,2,4]triazolo[4,3-a][1,4]diazépine-2-yl]-2-propynyl}phénanthridine-6(5H)-one ;

(R)-(2E,4E)-5-(4-méthoxyphényl)-N-[1-méthyl-4-(3-pyridinyl)butyl]-2,4-décadiènamide ;

iodure de 3-[4-[(R)-2-[(méthoxycarbonyl)oxy]-3-[(octadécylcarbamoyl)oxypropoxy]butyl]thiazolium ;

(2S,5S)-2-[(3-méthylsulfonyl-5-méthoxy-4-propyloxy)phényl]-5-(3,4,5-triméthoxyphényl)-tétrahydrofuranne,

et l'antagoniste du $LTD_4$ est choisi parmi l'ensemble comprenant :

acide (E)-4-[3-[2-(4-cyclobutyl-2-thiazolyl)éthényl]phénylamino]-2,2-diéthyl-4-oxobutanoïque ;

acide rac-6-acétyl-7-[[5-(4-acétyl-3-hydroxy-2-propylphénoxy)pentyl]oxy]-3,4-dihydro-2H-1-benzopyranne-2-carboxylique ;

sel de sodium du mono-N,N-diméthylamide de l'acide 5-(3-(2-(7-chloroquinoléine-2-yl)éthényl)-phényl)-4,6-dithianonanedicarboxylique.

4. Composition pharmaceutique selon la revendication 3, dans laquelle l'antagoniste du PAF est choisi parmi l'ensemble comprenant :

5-{3-[4-(2-chlorophényl)-9-méthyl-6H-thiéno[3,2-f][1,2,4]triazolo[4,3-a][1,4]diazépine-2-yl]-2-propynyl}phénanthridine-6(5H)-one ;

(R)-(2E,4E)-5-(4-méthoxyphényl)-N-[1-méthyl-4-(3-pyridinyl)butyl]-2,4-décadiènamide,

et l'antagoniste du $LTD_4$ est l'acide (E)-4-[3-[2-(4-cyclobutyl-2-thiazolyl)éthényl]phénylamino]-2,2-diéthyl-4-oxobutanoïque.

5. Composition pharmaceutique selon la revendication 4, dans laquelle l'antagoniste du PAF est la 5-{3-[4-(2-chlorophényl)-9-méthyl-6H-thiéno[3,2-f][1,2,4]triazolo[4,3-a][1,4]diazépine-2-yl]-2-propynyl}phénanthridine-6(5H)-one,

et l'antagoniste du $LTD_4$ est l'acide (E)-4-[3-[2-(4-cyclobutyl-2-thiazolyl)éthényl]phénylamino]-2,2-diéthyl-4-oxobutanoïque.

6. Composition pharmaceutique selon la revendication 4, dans laquelle l'antagoniste du PAF est le (R)-(2E,4E)-5-(4-méthoxyphényl)-N-[1-méthyl-4-(3-pyridinyl)butyl]-2,4-décadiènamide,

et l'antagoniste du $LTD_4$ est l'acide (E)-4-[3-[2-(4-cyclobutyl-2-thiazolyl)éthényl]phénylamino]-2,2-diéthyl-4-oxobutanoïque.

7. Utilisation d'une composition selon l'une quelconque des revendications 1 à 6 pour préparer des médicaments contre les troubles allergiques.